# EUROPÄISCHE PATENTSCHRIFT

(11) **EP 3 285 074 B1**
(45) Veröffentlichungstag und Bekanntmachung des Hinweises auf die Patenterteilung: **12.01.2022**
(21) Anmeldenummer: 17185313.8
(22) Anmeldetag: 08.08.2017
(51) Int. Cl.: G01N 35/10, B41J 2/21, B41J 2/045, B01L 3/02

(54) **MIKRODOSIEREINRICHTUNG UND AUTOMATISCHES MIKRODOSIERVERFAHREN**
MICRODOSING DEVICE AND AUTOMATIC MICRODOSING METHOD
DISPOSITIF DE MICRODOSAGE ET PROCÉDÉ DE MICRODOSAGE AUTOMATIQUE

(30) Priorität: 16.08.2016 DE 102016215240
(43) Veröffentlichungstag der Anmeldung: 21.02.2018
(73) Patentinhaber: Fraunhofer-Gesellschaft zur Förderung der angewandten Forschung e.V., 80686 München (DE)
(72) Erfinder: KLINGER, Michael, 72622 Nürtingen (DE); LASKE, Christopher, 70563 Stuttgart (DE)
(74) Vertreter: Schrell, Andreas

(56) Entgegenhaltungen:
- US-A1- 2007 086 021
- US-A1- 2010 265 287
- US-A1- 2015 116 406

## Beschreibung

Die vorliegende Erfindung betrifft Verfahren und Mittel zur kontrollierten automatischen Dosierung kleiner Flüssigkeitsmengen mit Mehrkanal-Mikrodosiereinrichtungen, geeignet zur Anwendung in automatischen Verfahren für biologische Assays und zur Kultivierung von Zellen und Geweben.

Die automatische Dosierung von kleinen Flüssigkeitsmengen mittels automatischer Mikrodosierroboter ist an sich bekannt. Mit solchen Anlagen werden flüssige Medien, so genannte "biologische Flüssigkeiten" wie Zellsuspensionen, Zellkulturmedien, Wirkstoffe und/oder Testsubstanzen, automatisch in Zielgefäße oder Substrate dosiert. Typische Anwendungen sind biologische Assays und die automatisierte Zell- und Gewebekultur ("single cell technology", "tissue engineering"). Üblicherweise sind die Zielgefäße zum Zwecke der Reihenuntersuchungen und für Hochdurchsatzverfahren Mehrfach-Zellkulturgefäße, so genannte Mikrotiter- oder Multiwellplatten, die meist im SBS-Format vorliegen. Diese weisen eine regelmäßige Anordnung von mehreren Einzelgefäßen ("wells"), meist 24 oder 96 auf einem gemeinsamen Träger auf. In bekannten Mikrodosierautomaten werden die einzelnen Zielgefäße über Pipettierroboter seriell adressiert oder alternativ parallel aus mehreren nebeneinanderliegenden Dosierkanälen. Jeder Dosierkanal wird durch eine separat aktuierbare Nadel oder Düse an der Mikrodosiereinrichtung gebildet.

Die grundsätzliche Funktionsweise solcher Mehrkanal-Mikrodosiereinrichtungen basiert darauf, dass die zu dosierende Flüssigkeitsmenge durch konstanten Druck oder durch einzelne Druckimpulse aus der Düse, Pipettenspitze oder Nadel verdrängt wird. Dabei löst sich jeweils ein Tropfen oder ein Flüssigkeitsstrahl von der Düsenspitze ab. Pipette, Düse oder Nadel sind im Gebrauch meist so über dem Zielgefäß positioniert, dass der Tropfen oder Strahl senkrecht nach unten fällt. Die I-DOT Nanodosiertechnologie ("immediate drop on demand technology", Fraunhofer-Institut für Produktionstechnik und Automatisierung IPA) ist ein Hochdurchsatzverfahren zur automatischen Prozessierung von Volumina ("liquid handling") im Nano- und Mikroliterbereich. Dabei werden Tropfen wiederkehrend direkt und berührungslos aus einer beispielsweise 96-Kanal Dosierplatte auf beliebige Zielsubstrate, besonders eine darunterliegende Mikrotiterplatte, dosiert. Als Dosierplatte dient eine beispielsweise 96-Well Mikrotiterplatte im SBS-Format, die sich durch jeweils eine kleine Bohrung im Wellboden auszeichnet. Die Bohrung bildet dabei jeweils die Dosierdüse. Der Durchmesser der Bohrung ist so gering, dass der Kapillardruck in der Bohrung höher ist als der hydrostatische Druck der Flüssigkeit im Well. Die Bohrung wirkt wie ein Schwellwertventil. Wird der Druck im Well durch einen auf das jeweilige Well druckdicht aufsetzbaren Aktuator (Dosierkopf) kurzzeitig stark erhöht, kann Flüssigkeit aus der Düse entweichen. Mit einem beispielsweise acht-fach parallelen Dosierkopf kann aus einer Reihe von nebeneinanderliegenden Wells der Vorlage durch deren Wellböden gebildeten dies aber wiederkehrend mit einer Wiederholrate von bis zu 600 Hz. Damit lassen sich Durchsätze in einem Volumenbereich von etwa 10 nl bis hin zu 100 µl realisieren.

Bei auf einer anderen Technologie basierenden Mehrkanal-Mikrodosiereinrichtungen (Multidrop-Mikrodosiersystem, z.B. Multidrop ^{™} Combi Reagenzien-Dispenser, Fa. Thermo Scientific), sind nebeneinanderliegende parallele Pipettenspitzen oder hohle Dosiernadeln ausgebildet, die jeweils über einen flexiblen Dosierschlauch gespeist werden. Jeder Dosierschlauch ist mit einer präzisen Peristaltikpumpe mit Schrittmotor verbunden, welche die zu dosierende Flüssigkeit in dem Dosierschlauch exakt fördert und dosiert. Pro ausgelöstem Dosierschritt wird zum Beispiel ein Volumen von 0.5 µl in Form eines Tropfens oder Strahls abgegeben, und zwar triggerbar wiederkehrend mit einer Wiederholrate von bis zu 20 Hz. Der zweckmäßige Dosierbereich liegt bei 0,5 µl bis etwa 5000 µl.

In der praktischen Anwendung hat sich nun aber gezeigt, dass an einzelnen Dosierkanälen, das heißt an Pipette oder Nadel oder Düse, bedingt durch Verunreinigungen oder aufgrund von Abnutzung oder Materialfehlern, Ungleichmäßigkeiten und Störungen im Dosiervorgang auftreten können. Dabei kann es vorkommen, dass Flüssigkeitstropfen nicht mehr sauber gebildet werden und sich Tropfen oder Flüssigkeitsstrahlen nicht mehr exakt in Idealrichtung, das heißt bevorzugt senkrecht, von der Dosierdüse lösen. Es kommt im Extremfall dazu, dass einzelne oder mehrere ausgelöste Dosierungen ganz ausbleiben oder dabei das Zielgefäß nicht oder mehr vollständig getroffen wird. Dies führt zu Fehldosierungen. Solche Dosierfehler lassen sich bisher nur durch manuelles und daher nur stichprobenhaftes Beobachten des Dosiervorgangs oder durch Nachkontrolle des Dosiererfolgs im Zielgefäß erkennen. Problematisch ist, dass in automatisierten Hochdurchsatzanlagen eine solche manuelle Kontrolle nicht praktikabel ist. Eine Fehldosierung muss daher bisher als systematische Unsicherheit mitberücksichtigt werden, was Kultivierungsergebnisse oder Untersuchungsergebnisse deutlich beeinflussen kann und Ausbeute und Effizienz der Gesamtanlage reduziert oder die Aussagekraft der biologischen Assays verschlechtert. Wünschenswert sind daher vollautomatische Systeme, welche Art und Qualität der Flüssigkeitsdosierung vollständig überwachen und beurteilen können. Idealerweise soll die Abweichung des Abgabewinkels des Flüssigkeitstropfens oder -Strahls von der Idealrichtung erkannt und günstigenfalls quantifiziert werden. Ebenso soll eine durchgehende qualitative und quantitative Überwachung der Flüssigkeitsabgabe an den Dosierkanälen geeignete Maßnahmen im laufenden Betrieb ermöglichen, ohne dass ein manueller Eingriff durch den Automatenbetreiber erforderlich wäre.

Aus der US 2007/086021 A1 ist eine Vorrichtung und ein Verfahren zur Bestimmung der Position eines aus einer Tintenstrahldrucker-ähnlichen Dosiereinrichtung abgegebenen Flüssigkeitstropfen bekannt, wobei in mindestens einer Lichtschrankeneinheit der Schattenwurf des Tropfens auf ein zweidimensionales Lichtdetektor-Array dieser Lichtschrankeneinheit ausgewertet wird.

Das der Erfindung zugrundeliegende technische Problem besteht in der Bereitstellung von Verfahren und Mitteln zur automatisierbaren wiederkehrenden Abgabe von Flüssigkeitstropfen oder Flüssigkeitsstrahlen in parallelen Mehrkanalsystemen, wobei die Art und Qualität der abgegebenen Flüssigkeit laufend automatisch kontrolliert werden und gegebenenfalls auf einen Sollwert kompensiert werden kann.

Das technische Problem wird vollständig gelöst durch die Bereitstellung einer Vorrichtung gemäß Anspruch 1 zur automatischen optischen Kontrolle von an Multikanal-Mikrodosiereinrichtungen abgegebenen Flüssigkeitstropfen oder Flüssigkeitsstrahlen. Diese enthält erfindungsgemäß insbesondere mindestens eine, bevorzugt genau eine, jedem Dosierkanal, das heißt Dosiernadel oder Dosierdüse der Mikrodosiereinrichtung, zugeordnete Lichtschrankeneinheit. Die Lichtschrankeneinheit besteht erfindungsgemäß aus mindestens einer, bevorzugt genau einer Lichtquelle mit inhomogenem Strahlprofil und mindestens einem, bevorzugt genau einem zugehörigen Lichtsensor mit einer Sensorfläche, auf welche der profilierte Lichtstrahl der Lichtquelle projiziert wird. Diese sind an dem Dosierkanal jeweils unmittelbar angeordnet, und zwar auf einem Träger. Dabei ist die Lichtschrankeneinheit so dimensioniert und so angeordnet, dass der profilierte Lichtstrahl zwischen Lichtquelle und Lichtsensor jeweils im Wesentlichen quer zur Ausbreitungsrichtung, das heißt zur Vorzugsrichtung der aus dem Dosierkanal abgegebenen Flüssigkeitstropfen oder Flüssigkeitsstrahlen verläuft. Weiter ist erfindungsgemäß dieser profilierte Lichtstrahl zumindest quer zur Ausbreitungsrichtung breiter dimensioniert als der dort durchtretende zu kontrollierende Flüssigkeitstropfen oder Flüssigkeitsstrahl.

Unter einem "profilierten Lichtstrahl" mit "inhomogenen Strahlprofil" wird im Rahmen dieser Erfindung ein kegelförmiges Lichtbündel mit einem insbesondere annähernd gaußschem oder leptokurtischem Intensitätsprofil verstanden (Gauß-Strahl). Es sind andere Intensitätsverteilungen denkbar, erfindungsgemäß ist ein Lichtkegel mit der höchsten Intensität in der optischen Achse, das heißt in der Mitte des kegelförmigen Lichtbündels, die zum Rand des Lichtbündels jeweils abfällt. Lichtquellen, welche ohne weiteres zur Erzeugung eines solchen Lichtkegels geeignet sind, sind bevorzugt ausgewählt aus der Gruppe lichtemittierenden Dioden (LED) und aus Halbleiter-Laserdioden. In einer ersten Variante ist dies ein im Wesentlichen planares LED-Leuchtfeld mit in an sich bekannter Art integrierter, das heißt in der Regel angegossener, Kondensorlinse. In alternativen Ausgestaltungen sind spezifische Mikrolinsen zur Strahlformung vorgesehen, um die Strahlprofile zu erhalten; diese sind bevorzugt ausgewählt aus asphärischen Linsen und Axicon-Linsen.

Erfindungsgemäß projiziert der profilierte Lichtstrahl auf eine Sensorfläche des gegenüberliegenden Lichtsensors, welche das gesamte Strahlprofil des auftreffenden Lichtstrahls erfasst und dessen Intensität über seine Ausdehnung in der Projektionsfläche integriert. Der Lichtsensor erzeugt so einen Sensorstrom, welcher der integrierten Strahlintensität entspricht.

Der abgegebene Flüssigkeitstropfen oder Flüssigkeitsstrahl durchbricht den Lichtstrahl der erfindungsgemäß unmittelbar an dem Dosierkanal angeordneten Lichtschrankeneinheit, wodurch der Lichtsensor an seiner mit dem profilierten Lichtstrahl beleuchteten Sensorfläche einen Abfall in der Lichtintensität registriert. Der Abfall des Lichtstroms, das heißt der Grad der Abschattung am Sensor, ist dabei abhängig von der Stelle innerhalb des profilierten Lichtstrahls, an welcher der Tropfen oder Strahl diesen unterbricht. In der erfindungsgemäßen Ausgestaltung des Lichtstrahls mit insbesondere gaußschem oder vergleichbar profilierten Intensitätsprofil erfolgt die maximale Abschattung, das heißt die maximale Verminderung des Lichtstroms dann, wenn der Lichtstrahl gerade in seiner Achse, das heißt in der Mitte, unterbrochen wird. Diese maximale Abschattung kennzeichnet in der bevorzugten Ausgestaltung des Verfahrens die Idealrichtung oder Sollposition des abgegebenen Flüssigkeitstropfens oder -strahls.

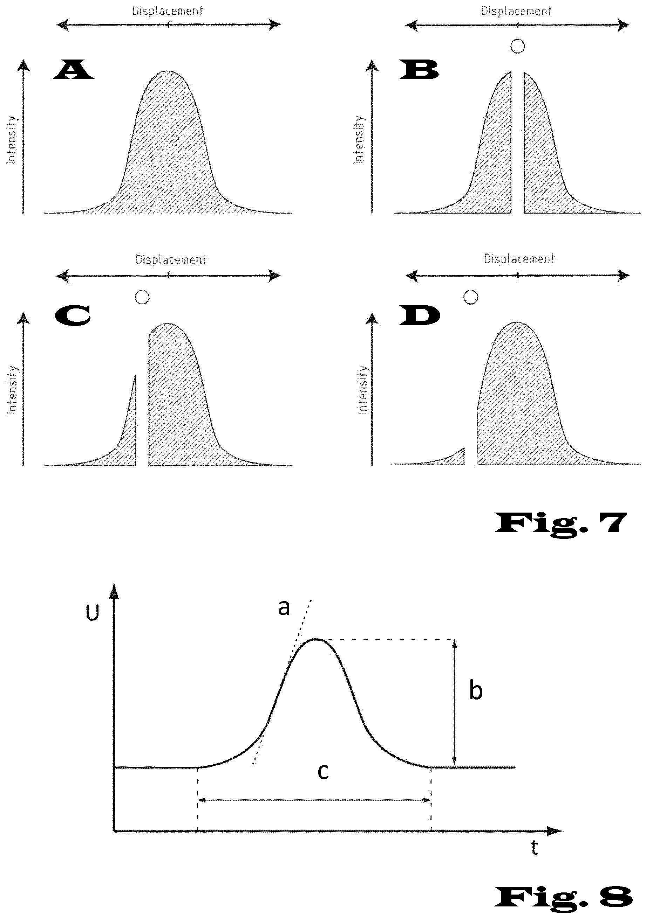

Ohne an die Theorie gebunden sein zu wollen, erlaubt diese Lichtschrankeneinheit eine einfache und gleichzeitig hinreichend genaue Überwachung der Position und Richtung des abgegebenen Flüssigkeitstropfens oder Flüssigkeitsstrahls und der Qualität der Flüssigkeitsabgabe. Die durch eine programmierte Auswerteeinheit (Analogrechner oder digitaler Programmrechner) ist eine Bewertung der Abweichung der Art und Qualität der Flüssigkeitsabgabe von einem gewünschten Ideal über die zeitliche Änderung des Sensorstroms bei Durchtritt des Flüssigkeitstropfens oder Flüssigkeitsstrahls durch den Lichtstrahl möglich. Diese Analyse gestaltet sich im einfachsten Fall wie folgt: Je weiter der Flüssigkeitstropfen oder Flüssigkeitsstrahl von der idealen Mittenposition (Idealrichtung) abweicht, desto geringer fällt die Änderung des Sensorstroms aufgrund der Abschattung des profilierten Lichtkegels aus. Dieser Funktionszusammenhang ist in den Figuren 7A bis 7D graphisch erläutert.

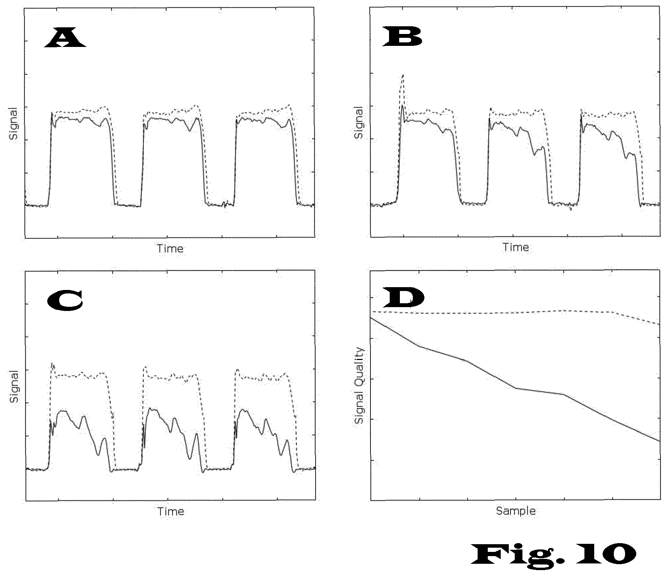

Gleichzeitig erlaubt die erfindungsgemäße Anordnung in der Lichtschrankeneinheit die Bestimmung der Qualität des abgegebenen Flüssigkeitstropfens oder insbesondere Flüssigkeitsstrahl, wenn der zeitliche Verlauf des Sensorstroms während der Abschattung des Lichtstrahls der Lichtschrankeneinheit durch den Flüssigkeitstropfen oder -strahl registriert wird: Bei Strahlstörungen, die zu Fehldosierungen führen können, kommt es zu signifikanten Abweichungen im erwarteten Signalverlauf. Dies ist beispielsweise in den Figuren 10A bis 10C illustriert; sie zeigen verschiedene funktionsgestörte Dosierkanäle. Der Parameter der erfindungsgemäßen Signalanalyse ist hier besonders die zeitliche Signalvarianz des Sensorsignals, insbesondere zu den Zeitintervallen, in denen die dosierten Tropfen oder Strahlen die Lichtschrankeneinheit(en) durchlaufen. Eine größere Signalvarianz zeigt eine Strahlstörung an, zum Beispiel ungleichmäßige Strahlform oder Strahlrichtung (unterbrochener Strahl, zu breiter Strahl, abgelenkter Strahl) oder eine ungleichmäßige Tropfenbildung (zu großer, ungleichmäßiger oder "aufbrechender" Tropfen).

In einer bevorzugten Ausführung ist jedem Dosierkanal, das heißt jeder Nadel oder Düse der Mikrodosiereinrichtung jeweils genau eine Lichtschrankeneinheit, bestehend aus einer Lichtquelle und einem Lichtsensor unmittelbar zugeordnet, das heißt besonders in einer Multikanal-Mikrodosiereinrichtung mit mehreren, vorzugsweise in regelmäßigen Reihen (eindimensionales Array) oder Gittern (zweidimensionales Array) angeordneten Dosierkanälen ist jedem einzelnen Dosierkanal eine einzige Lichtschrankeneinheit, bestehend aus Lichtquelle und Lichtsensor unmittelbar zugeordnet. Dabei ist die Lichtschrankeneinheit zusammen auf einem gemeinsamen Träger angeordnet, bevorzugt sind mehrere solche Lichtschrankeneinheiten nebeneinander und zueinander bevorzugt regelmäßig beabstandet in einer Reihe oder in einem Gitter angeordnet. Vorzugsweise ist der Träger unmittelbar an den Enden der Dosierkanäle, das heißt Nadeln oder Düsen der Mikrodosiereinrichtung angeordnet. Dies erlaubt eine kompakte und gleichzeitig mechanisch stabile Ausgestaltung einer integralen "Sensorplatte" aus einer Vielzahl von Lichtschrankeneinheiten, die zusammen auf einem gemeinsamen Träger angeordnet sind. Bevorzugt weist der Träger oder die Trägerplatte jeweils ein Fenster auf, wo der Flüssigkeitstropfens oder -strahl, der aus der Düse oder Nadel austritt, hindurchtreten kann.

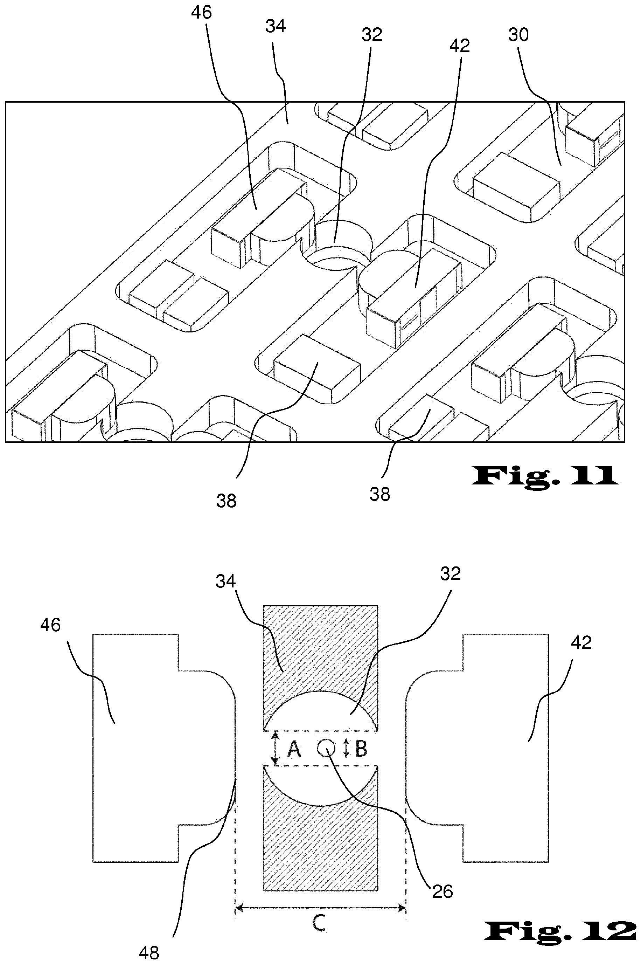

In bevorzugter Ausgestaltung ist pro Lichtschrankeneinheit zwischen Lichtquelle und Lichtsensor eine strahlformende Apertur oder Blende angeordnet zum Zwecke der Abschattung nicht benötigter Strahlanteile, vornehmlich zur Verbesserung der Signalqualität des Sensorsignals. Die Blende blendet den aus der Lichtquelle austretenden Lichtkegel soweit ab, dass gerade ein Lichtkegel erhalten bleibt, der die Sensorfläche des Lichtsensors abdeckt, wobei die Breite des Lichtkegels an der Stelle des Durchtritts der abgegebenen Flüssigkeit stets größer ist als die Breite oder Abmessung des durchtretenden Flüssigkeitstropfens oder -strahls (siehe Figur 12). Die Blende kann dabei als Teil des Gehäuses oder Trägers ausgebildet sein, worauf die Lichtschrankeneinheit jeweils angeordnet ist. Dies erlaubt eine kompakte und gleichzeitig mechanisch stabile Ausgestaltung der integralen "Sensorplatte".

Abweichungen von der Idealrichtung der Flüssigkeitstropfen oder -strahlen in Richtung der Achse des Lichtstrahls können durch eine einzelne Intensitätsmessung, nicht ohne weiteres erkannt werden. Durch intelligenten Vergleich mehrerer Messwerte und unter der Annahme, dass die überwiegende Zahl der Flüssigkeitstropfen oder -Strahlen in Idealposition abgegeben werden, sind aber Rückschlüsse auf eine Fehlpositionierung auch in Längsrichtung des Lichtstrahls möglich. Dies deshalb, da erfindungsgemäß der Lichtstrahl divergiert und eine kegelförmige Ausbreitung hat. In bevorzugter Ausgestaltung weist die Vorrichtung jedoch zumindest zwei unabhängige Lichtschrankeneinheit pro Dosierkanal auf, deren Lichtstrahlen zueinander in einem Winkel stehen, um zwei Raumrichtungen der Positionsabweichung von Flüssigkeitstropfen oder -strahlen genauer erfassen zu können. In dieser Variante sind jedem Dosierkanal unmittelbar zumindest zwei, bevorzugt genau zwei, separate Lichtschrankeneinheiten zugeordnet, wobei die beiden Lichtschrankeneinheiten jeweils aus je einer Lichtquelle und je einem Lichtsensor bestehen. Die beiden Lichtschrankeneinheiten sind so zueinander angeordnet, dass deren Lichtstrahlen, zumindest in der Ebene quer zur Ausbreitungsrichtung oder Idealrichtung der zu kontrollierenden Flüssigkeitstropfen oder Flüssigkeitsstrahlen, in einem Winkel zueinander stehen. Bevorzugt beträgt der Winkel der Lichtstrahlen zueinander in dieser Ebene etwa 90°, alternativ bevorzugt etwa 60°. In weiteren Varianten beträgt der Winkel der Lichtstrahlen zueinander (in dieser Ebene) etwa 30° bis 150°, bevorzugt 45° bis 135°, weiter bevorzugt von 60° bis 120°.

In einer bevorzugten Ausgestaltung enthält die erfindungsgemäße Vorrichtung zum Zwecke der Auswertung und insbesondere zur Steuerung oder Regelung des Abgabeverhaltens des jeweiligen Dosierkanals der Mikrodosiereinrichtung eine programmierte Auswerteeinheit, die zumindest mit dem jeweiligen Lichtsensor verbunden ist und geeignet ist, mittels entsprechender Strom-Spannungswandler und Messverstärker, Hoch- und/oder Bandpassfilter, ein prozessiertes, konditioniertes und vorzugsweise zeitlich aufgelöstes Sensorsignal zur erhalten, welches die integrierte Intensität des auf die jeweilige Sensorfläche fallenden Strahlprofils repräsentiert. Auswertbare Signalparameter sind das Signalprofil: Pulslänge, Pulshöhe, Pulsflanke (Steigung), die Wiederholrate, das Frequenzspektrum, besonders hochfrequente Anteile, ermittelt vorzugsweise über geeignete Filter oder durch Fouriertransformation, und die statistische Signalvarianz. Ebenfalls erfassbar ist der Offset oder Bias, der durch längerfristige Drift an den Sensoren oder Dejustierung des Lichtwegs an den Lichtschrankeneinheiten auftreten kann. Dieser ist gegebenenfalls kompensierbar durch Filter in der analogen Messkette und/oder durch gleitende Offsetkompensation.

Die programmierte Auswerteeinheit enthält vorzugsweise zusätzlich eine Recheneinheit mit Speichereinheit zur Ermittlung und Bewertung des zeitlichen Verlaufs der Lichtintensität an der Sensorfläche und/oder zur Ermittlung der Differenz der Intensität an der Sensorfläche, einmal zum Zeitpunkt des Durchtritts des Flüssigkeitstropfens oder Flüssigkeitsstrahls durch den Lichtstrahl und einmal zu dem Zeitpunkt unmittelbar vor oder nach diesem Ereignis (ungestörter Lichtstrahl). Weiter besitzt die Auswerteeinheit bevorzugt eine Signal- oder Steuereinrichtung zur Signalisierung der Abweichung des zeitlichen Intensitätsverlaufs der an dem Sensor gemessenen Lichtintensität von einem vorbestimmbaren oder vorgespeicherten "idealen" Intensitätsverlauf und/oder zur Signalisierung der Abweichung von einer vorbestimmbaren, vorgespeicherten Intensitätsdifferenz und/oder zur Erzeugung eines geeigneten Steuersignals, besonders zur Kompensation einer identifizierten Regelabweichung.

Dabei ist die Auswerteeinheit, insbesondere in Verbindung mit der Recheneinheit und Speichereinheit, so programmiert, dass zur Kontrolle der Position des jeweils an den Kanälen abgegebenen Flüssigkeitstropfens oder -strahls nach den hierin beschriebenen Kriterien der mittlere maximale Hub des prozessierten Sensorsignals, das heißt des Intensitätsverlaufs, beim Durchtritt des Flüssigkeitstropfens oder - strahls durch den jeweiligen Lichtstrahl die Idealposition des Flüssigkeitstropfen oder -strahls anzeigt und eine jeweilige Verminderung des Amplitudenhubs unter einen vorbestimmbaren Schwellwert eine Abweichung oder Richtungsänderung des abgegebenen Flüssigkeitstropfens oder -strahls von dieser Idealposition anzeigt.

Alternativ oder zusätzlich ist die programmierte Auswerteeinheit, besonders in Verbindung mit der Recheneinheit und Speichereinheit, so programmiert, dass zur Kontrolle der Qualität des jeweils an den Dosierkanälen abgegebene Flüssigkeitstropfens oder -strahls nach den hierin beschriebenen Kriterien:
der mittlere maximale Hub des prozessierten Sensorsignals, das heißt des Intensitätsverlaufs, beim Durchtritt des Flüssigkeitstropfens oder -strahls durch den jeweiligen Lichtstrahl der Lichtschrankeneinheit und/oder
die minimale Signalvarianz des prozessierten Sensorsignals, das heißt des Intensitätsverlaufs zu diesem Zeitpunkt
   eine Idealqualität des Flüssigkeitstropfens oder -strahls anzeigt und
eine Verminderung des Amplitudenhubs unter einen vorbestimmbaren Schwellwert oder
ein Ausbleiben des Amplitudenhubs oder
eine Erhöhung der Signalvarianz des prozessierten Sensorsignals über einen vorbestimmbaren Schwellwert
   bei Auslösen der Abgabe des Flüssigkeitstropfens oder -strahls an der Mikrodosiereinrichtung eine Fehldosierung an dem jeweiligen Dosierkanal anzeigt.

In einer bevorzugten Ausgestaltung weist die erfindungsgemäße Vorrichtung zusätzlich zumindest eine Signal-Rückleitung auf, worin die programmierte Auswerteeinheit, insbesondere über die Signal- oder Steuereinrichtung, mit einem Stellglied an der Mikrodosiereinrichtung verbunden ist, zur Regelung der Abgabe des Flüssigkeitstropfens oder -strahls aus der Mikrodosiereinrichtung anhand voreingestellter Werte, welche die Idealposition oder Idealqualität eines abgegebenen Flüssigkeitstropfens oder Flüssigkeitsstrahls bedeuten. Die Regelung dient der automatischen Kompensation von systembedingten Abweichungen der Flüssigkeitsabgabe an den jeweiligen Dosierkanälen, Nadeln oder Düsen im laufenden Betrieb durch geeignete Stellglieder. Geeignete Stellgrößen sind Amplitude und/oder Druck an dem jeweiligen Aktor oder Dosierkopf, welcher die Flüssigkeitsabgabe an dem Dosierkanal auslöst. Eine alternative oder zusätzliche Stellgröße ist die Frequenz der Auslösung der Flüssigkeitsabgabe. Eine alternative oder zusätzliche Stellgröße ist die Dauer der Auslösung der Flüssigkeitsabgabe. Eine alternative oder zusätzliche Stellgröße ist die Düsengeometrie. Eine alternative oder zusätzliche Stellgröße ist eine auslösbare Maßnahme zur Düsen- oder Nadelreinigung, beispielsweise über zusätzlich auslösbare Druckwellen, welche eventuell angesammelte Verunreinigungen oder Verstopfungen der Düse oder Nadel austreiben. Geeignete Aktoren und Stellglieder sind bevorzugt piezoelektrische Aktoren, alternativ bevorzugt sind elektromagnetische Aktoren.

Ein wesentlicher Aspekt der vorliegenden Erfindung ist dabei auch der konstruktiv einfache integrale Aufbau der jeweiligen Lichtschrankeneinheiten an einem gemeinsamen Träger, was die Reinigbarkeit, Austauschbarkeit und gleichzeitig die Präzision der Messung erleichtert. Es wird bewusst auf komplizierte optische Vorrichtungen verzichtet. Durch die intelligente Programmierung der Auswerteeinheit in Verbindung mit der Lichtschrankeneinheit mit profiliertem Lichtstrahl und integrierender Sensorfläche wird ein einfaches und dennoch präzises Messinstrument bereitgestellt, welches die Qualität der Flüssigkeitsdosierungen an den jeweiligen Dosierkanälen zuverlässig bestimmen, diese anzeigen und insbesondere durch geeignete Rückleitung auf einen Sollzustand kompensieren kann.

Diese Ausführung erlaubt vorteilhafterweise die Bereitstellung einer vollautomatischen autonomen, insbesondere selbst adaptierenden Mikrodosiereinrichtung, welche einen unterbrechungsfreien vollautomatischen Dosierbetrieb ermöglicht. Dies ist insbesondere von Bedeutung in Anlagen zur automatischen Kultivierung von Zellen und Geweben, in welchen schon allein aufgrund der einzuhaltenden Sterilität und Reinheit, aber auch aufgrund von einzuhaltenden Betriebsbedingungen, insbesondere Umgebungsluft, Begasung und Temperatur, Eingriffe durch den Automatenbetreiber stets zu vermeiden wären. Die erfindungsgemäße optische Kontrollvorrichtung erlaubt daher die Bereitstellung von Multikanal-Mikrodosiereinrichtungen, welche wiederkehrend kleine Mengen an Flüssigkeiten, besonders Flüssigkeitstropfen oder Flüssigkeitsstrahlen kontrolliert und in gleichbleibender Qualität geben können, und zwar in unterbrechungsfreiem automatisierten Betrieb.

Demgemäß ist ein weiterer Gegenstand dieser Erfindung eine verbesserte Multikanal-Mikrodosiereinrichtung zur automatischen Dosierung von Flüssigkeit im Nanoliter oder Mikroliterbereich mittels wiederkehrender Abgabe von Flüssigkeitstropfen oder Flüssigkeitsstrahlen kleinen Volumens, die geeignet ist für den unterbrechungsfreien automatischen Betrieb. Diese enthält die optische Kontrollvorrichtung der vorliegenden Erfindung als integralen Bestandteil, besonders als unmittelbar an den Dosierkanälen angeordnete integrale Sensorplatte mit mehreren auf einem gemeinsamen Träger geträgerten Lichtschrankeneinheiten.

Ein weiterer Gegenstand der Erfindung ist ein Verfahren zur kontrollierten intermittierenden Abgabe von Flüssigkeitstropfen oder Flüssigkeitsstrahlen an oder mittels einer Multikanal-Mikrodosiereinrichtung, insbesondere unter Verwendung der optischen Kontrollvorrichtung gemäß dieser Erfindung. Das Verfahren enthält zumindest folgende Schritte: In einem ersten Schritt wird eine kurzfristige Abgabe jeweils eines Flüssigkeitstropfens oder -strahls geringen Volumens an den jeweiligen Dosierkanälen, Nadeln oder Düsen der Mikrodosiereinrichtung ausgelöst.

In einem weiteren Schritt werden die Lichtintensitäten an der über einen Lichtstrahl mit inhomogenen Strahlprofil beleuchteten Sensorfläche eines Lichtsensors, welcher jeweils den Kanälen der Mikrodosiereinrichtung zugeordnet ist, im zeitlichen Verlauf beim Durchtreten der abgegebenen Flüssigkeit durch den jeweiligen Lichtstrahl registriert. In einem weiteren Schritt findet ein automatisches Bewerten der über die Zeit registrierten Lichtintensitäten anhand vorgegebener Intensitätswerte oder Intensitätsverläufe statt. In einem weiteren Schritt wird zumindest ein Steuersignal oder Warnsignal abgegeben, welches zur Regelung der Abgabe der Flüssigkeit aus der Mikrodosiereinrichtung in einem geschlossenen Regelkreis und/oder zur Signalisierung einer Fehldosierung an dem jeweiligen Kanal der Mikrodosiereinrichtung dient.

Gegenstand der Erfindung ist damit besonders ein Verfahren zur kontrollierten Abgabe von Flüssigkeitstropfen oder -strahlen an Dosierkanälen einer Multikanal-Mikrodosiereinrichtung, enthaltend die Schritte:
- Auslösen einer kurzfristigen Abgabe jeweils eines einzelnen Flüssigkeitstropfens oder -strahls an den Dosierkanälen,
- Beleuchten des jeweils abgegebenen Flüssigkeitstropfens oder -strahls mit einem Lichtstrahl mit inhomogenem Strahlprofil und Registrieren der jeweiligen Abschwächung der über die Breite des Lichtstrahls integrierten Intensität bei Abschattung des Lichtstrahls durch diesen Flüssigkeitstropfen oder -strahl, mit der Lichtschrankeneinheit, ausgestaltet gemäß der vorstehend beschriebenen Erfindung,
- automatisches Bewerten des jeweils registrierten abgabekorrelierten Intensitätsverlaufs und
- Ausgeben eines Steuer- oder Warnsignals als Ergebnis der Bewertung zum Zwecke der Regelung der Abgabe der Flüssigkeit aus dem jeweiligen Dosierkanal und/oder zur Signalisierung von Fehldosierungen.

Die Bewertung erfolgt dabei bevorzugt mit der Maßgabe, dass die mittlere maximale Amplitude der abgabekorrelierten Intensitätsabschwächung bei Auslösen der Abgabe des einzelnen Flüssigkeitstropfens oder -strahls die Idealdosierung anzeigt, wohingegen
- die Verringerung der abgabekorrelierten Intensitätsabschwächung unter einen vorbestimmten Schwellwert,
- das Ausbleiben einer abgabekorrelierten Intensitätsabschwächung und/oder
- die Erhöhung der statistischen Signalvarianz eines abgabekorrelierten Intensitätsverlaufs über einen vorbestimmten Schwellwert
   jeweils eine Fehldosierung an dem jeweiligen Dosierkanal anzeigt.

Bevorzugt ist zur Kontrolle der jeweils an den Kanälen abgegebenen Flüssigkeitstropfen oder -strahlen, das heißt besonders von deren Abweichung von einer Idealrichtung, vorgesehen, dass der maximale Amplitudenhub des Intensitätssignals bei Durchtritt der abgegebenen Flüssigkeit durch den jeweiligen Lichtstrahl die Idealrichtung des Flüssigkeitstropfens oder -strahls anzeigt und eine Verringerung des Amplitudenhubs, insbesondere gegenüber eines gemittelten maximalen Amplitudenhubs, eine Abweichung der Position oder Richtung des Flüssigkeitstropfens oder -strahls davon anzeigt.

In einer Variante ist zur Kontrolle der Qualität des jeweils an den Kanälen abgegebenen Flüssigkeitstropfens oder -strahls, das heißt besonders von dessen Abweichung von einer Idealqualität, vorgesehen, dass eine Abweichung des Amplitudenhubs des Intensitätssignals bei Durchtritt der abgegebenen Flüssigkeit durch den jeweiligen Lichtstrahl, insbesondere gegenüber eines gemittelten maximalen Amplitudenhubs, und insbesondere die zeitweise Verminderung des Amplitudenhubs oder eine fluktuierende Signalamplitude oder ein zeitweises Ausbleiben eines Amplitudenhubs der Intensität beim Auslösen der Flüssigkeitsabgabe eine Fehldosierung an dem jeweiligen Kanal anzeigt.

Die Erfindung wird durch die nachfolgenden Ausführungsbeispiele und Figuren näher erläutert:

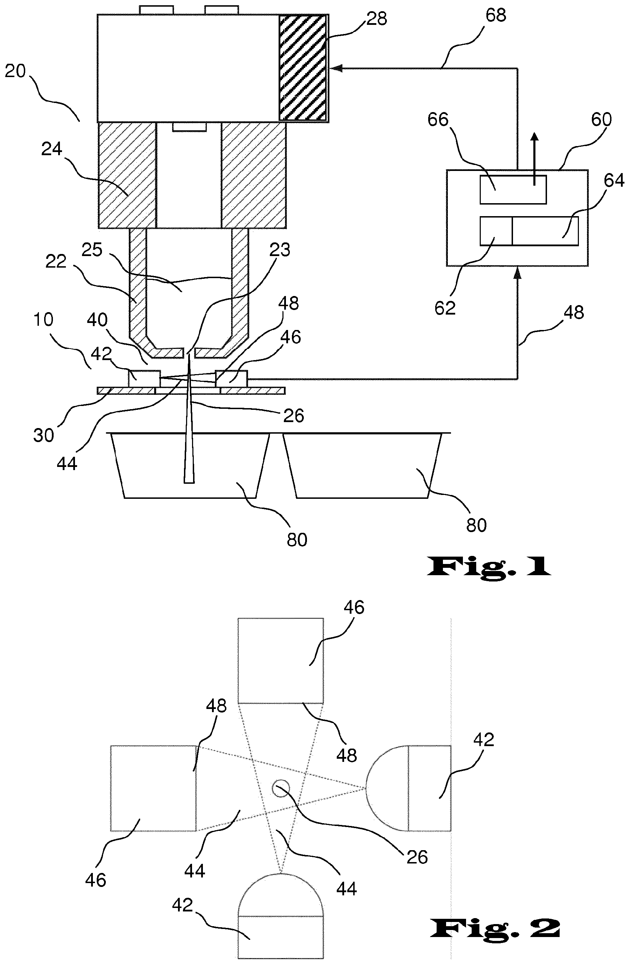

Figur 1 zeigt eine schematische Darstellung des Gesamtaufbaus einer Mikrodosiereinrichtung mit optischer Kontrollvorrichtung. Es ist zur Veranschaulichung einer der mehreren Dosierkanäle gezeigt. Die Mikrodosiereinrichtung (20) weist pro Dosierkanal (22) einen Treiber oder Dosierkopf (24) auf. Der Dosierkopf ist geeignet, wiederkehrende Druckimpulse auf die vorgelegte Flüssigkeit (25) des Dosierkanals (22) auszuüben, um die Flüssigkeit aus der Düse (23) auszutreiben. Unmittelbar unterhalb des Auslasses oder Düse (23) des Dosierkanals (22) ist die erfindungsgemäße optische Anordnung (10) positioniert. Diese besteht im Wesentlichen aus einer geträgerten Lichtschrankeneinheit (40) mit einer Lichtquelle (42) zur Erzeugung eines profilierten Lichtstrahls (40), welcher auf die Sensorfläche (48) eines Sensors (46) projiziert. Lichtquelle (42) und Sensor (46) bilden zusammen die Lichtschrankeneinheit (40), welche für jeden Dosierkanal (22) der Mikrodosiereinrichtung (20) zumindest jeweils einmal vorhanden ist. Die Lichtschrankeneinheiten (40) sind auf dem gemeinsamen Träger (30) angeordnet und bilden, insbesondere in mehrkanaliger Ausführung, die erfindungsgemäße optische Vorrichtung (10). Der Lichtstrahl (44) der Lichtschrankeneinheit (40) ist jeweils so orientiert, dass er von den aus der Mikrodosiereinrichtung (20) abgebbaren Flüssigkeitstropfen oder Flüssigkeitsstrahl (26) querend unterbrochen wird. Dabei ist vorgesehen, dass der profilierte Lichtstrahl (44) an dieser Stelle breiter, das heißt ausgedehnter ist, als der durchlaufende Flüssigkeitstropfen oder Flüssigkeitsstrahl (26). Der Flüssigkeitstropfen oder -strahl (26) verläuft vorzugsweise senkrecht in vertikaler Richtung dem Schwerkraftvektor folgend. Die aus der Mikrodosiereinrichtung (20) abgebbare Flüssigkeit (25) wird in die Wanne oder Vertiefung (80) dosiert. Diese ist vorzugsweise Bestandteil einer Mikrotiterplatte oder Multiwellplatte mit mehreren nebeneinander regelmäßig angeordneten Wannen oder Vertiefungen (80). Das Signal des Sensors (46) jeder Lichtschrankeneinheit (40) wird über je eine Leitung (48) einer Auswerteeinheit (60) zugeführt, die so programmiert ist, dass die Abweichung der Lichtintensität an der Sensorfläche des Sensors (46) bei Durchlaufen des Flüssigkeitstropfens oder -Strahls (26) durch den profilierten Lichtstrahl (44) durch Vergleich mit vorher erhobenen Intensitätswerten und/oder durch Vergleich mit voreingestellten Intensitätswerten oder zeitlichen Intensitätsverläufen zur Beurteilung der Qualität, der Dosierrichtung, und/oder von Fehldosierungen herangezogen werden kann. Dazu weist die programmierte Auswerteeinheit (60) vorzugsweise eine Recheneinheit (64) mit zugeordneter Speichereinheit (62), welche die Messwerte der Lichtintensität speichert, auf. Diese ist mit einer Signaleinrichtung (66) verbunden, die spezifisch geeignet ist, die Abweichung von einem vorbestimmbaren Idealwert, besonders einem vorbestimmbaren zeitlichen Intensitätsverlauf oder vorbestimmbarer Intensitätsdifferenz eine Fehldosierung zu signalisieren. Bevorzugt ist eine Rückleitung (68) vorgesehen, über welche mindestens ein Regelsignal (Stellgröße) von der programmierten Auswerteeinheit (60) auf mindestens ein Stellglied (28) an der Mikrodosiereinrichtung (20) übermittelt wird, welches eines oder mehrere Parameter der Flüssigkeitsdosierung des jeweiligen Dosierkanals (22) steuert und insbesondere in Abhängigkeit von der detektierten Qualität der Flüssigkeitsabgabe automatisch regelt. In der dargestellten Ausführung ist das Stellglied (28) eine Drucksteuereinrichtung, welche die Amplitude und Frequenz der Druckbeaufschlagung bei der Abgabe der Flüssigkeit (25) aus dem Dosierkanal (22) bestimmt.

Figur 2 zeigt eine schematische Darstellung eines Paars erfindungsgemäßer Lichtschrankeneinheiten (40) in Blickrichtung der abgegebenen Flüssigkeit (26). Eine Lichtquelle (42) erzeugt jeweils einen Strahlkegel (44) mit einem inhomogenen, insbesondere gaußschen Intensitätsprofil, welcher auf die Sensorfläche (48) eines gegenüber liegenden Sensors (46) projiziert wird. Zusammen mit einer zweiten dazu identischen Lichtschrankeneinheit (40) sind zwei im Wesentlichen senkrecht gekreuzt verlaufenden Lichtstrahlen (44) ausgebildet zur verbesserten Positionsbestimmung des durchlaufenden Flüssigkeitstropfens oder -strahls (26).

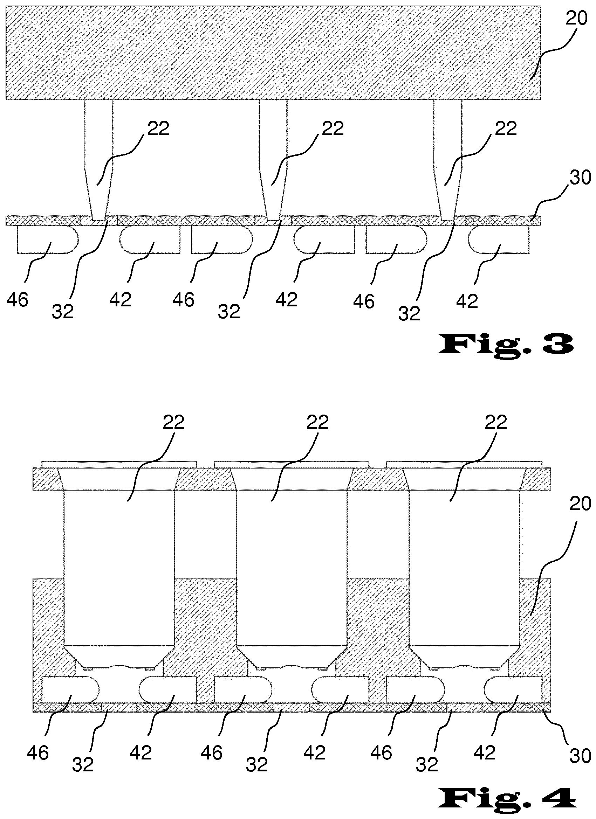

Figur 3 und Figur 4 zeigen jeweils schematische Darstellungen spezifischer Ausgestaltungen von Mehrkanal-Mikrodosiereinrichtungen (20) mit jeweils mehreren nebeneinanderliegenden parallel zu betreibenden Dosierkanälen (22) in Form von Düsen oder Nadeln. Unmittelbar an der Spitze jedes Dosierkanals (22) ist jeweils mindestens eine Lichtschrankeneinheit, bestehend aus Lichtquelle (42) und Lichtsensor (46), jeweils zusammen auf einem Träger (30) angeordnet. Der Träger (30) ist in den dargestellten Ausführungen als durchgehende Platte ausgeführt, welche jeweils an den Stellen des Durchtritts der abgegebenen Flüssigkeitstropfen oder -strahlen ein Fenster (32) aufweist.

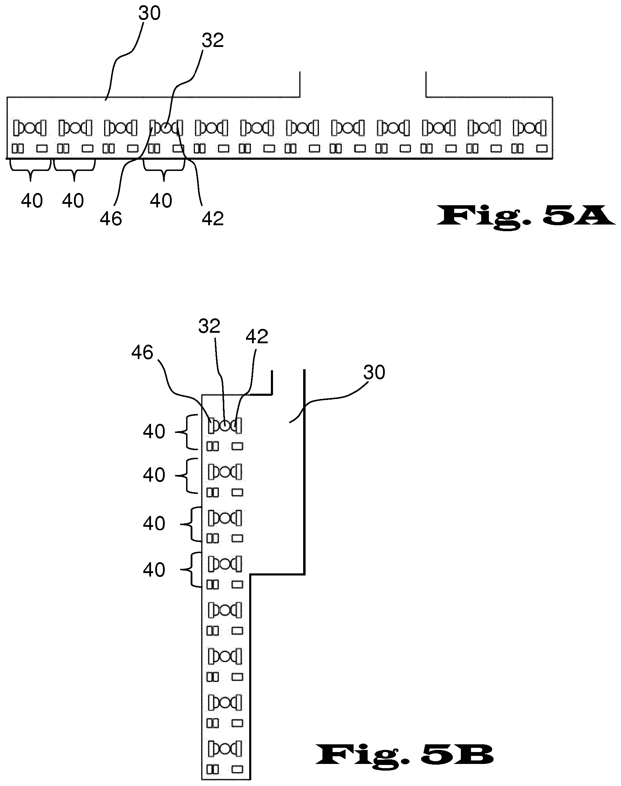

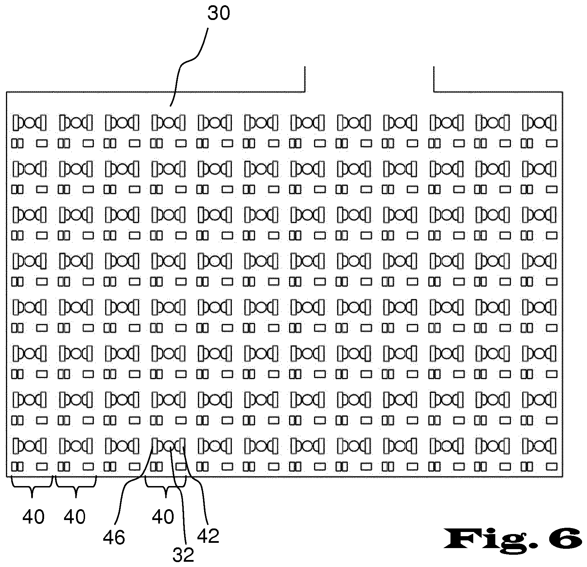

Figuren 5A und 5B und Figur 6 zeigen jeweils schematische Draufsichten on Varianten erfindungsgemäßer Vorrichtungen in bevorzugter Ausführung als integrierte Sensorplatten mit mehreren parallel zu betreibenden, zueinander regelmäßig beanstandeten Lichtschrankeneinheiten (40), jeweils bestehend aus einer Lichtquelle (43) und einem zugehörigen Lichtsensor (46), auf einem gemeinsamen Träger (30) und mit dazwischen liegenden Fenster (32) zum Durchtritt abgegebener Flüssigkeitstropfen oder -strahlen.

Figur 7 zeigt die Intensitätsverteilung im Strahlprofil des profilierten Lichtstrahls der erfindungsgemäßen Vorrichtung entlang der Querschnittslinie an der Sensorfläche eines Lichtsensors. Dabei ist in der Sensoranordnung bevorzugt vorgesehen, dass die Strahlintensität über die gesamte Breite des auftreffenden Lichtkegels integriert wird. Die gemessene Lichtintensität entspricht dem Integral unter der Fläche der dargestellten Kurve. Bei undurchbrochenem Lichtstrahl (keine Abgabe von Flüssigkeit oder ausbleibende Abgabe von Flüssigkeit) wird die höchste Lichtintensität ("intensity") registriert (Figur 7A). Durchbricht abgegebene Flüssigkeit den profilierten Lichtstrahl genau in der optischen Achse, das heißt mittig (=Idealposition), ist die größte Abschwächung der Lichtintensität zu beobachten (Figur 7B). Mit zunehmender Abweichung ("displacement") des Flüssigkeitstropfens von der Idealposition fällt die zeitweise Abschattung, das heißt die Intensitätsverringerung bei Durchlaufen des Flüssigkeitstropfens durch den profilierten Lichtstrahl, geringer aus (Figur 7C und 7D), was zur Positionsbestimmung und Qualitätsbeurteilung herangezogen wird.

Figur 8 zeigt wesentliche Kenngrößen, die in der programmierten Auswerteeinheit der erfindungsgemäßen Vorrichtung im zeitlichen Verlauf des jeweiligen Sensorsignals zur Bestimmung der Qualität der Flüssigkeitsdosierung herangezogen werden. Dargestellt ist der zeitliche Verlauf der Signalspannung proportional zum invertierten Sensorstrom: eine höhe Spannung zeigt eine geringere Strahlungsintensität an der Sensorfläche an. Beim Durchbrechen des Lichtstrahls durch den abgegebenen Flüssigkeitstropfens oder -strahls ist ein Spannungsanstieg zu verzeichnen. Steilheit und zeitlicher Verlauf des Spannungsanstiegs (a) gibt unmittelbar Aufschluss über die Qualität der Flüssigkeitsdosierung, insbesondere bei Abgabe von Flüssigkeitsstrahlen kann hier die Qualität, insbesondere Gleichmäßigkeit des Flüssigkeitsstrahls beurteilt werden. Die Höhe (b) der Signalamplitude zeigt insbesondere, wie in Figur 7A bis 7D dargestellt, die Position des abgegebenen Flüssigkeitstropfens oder -strahls an. Gleichzeitig lassen sich, bei ausreichender Kenntnis oder Grundannahme über die Signalamplitude (b) von korrekten Dosierungen in Idealrichtung, anderweitige Aussagen über die Qualität der Flüssigkeitsabgabe treffen. Hierzu zeigen auch der Verlauf und die Länge (c) der Signaländerung, Art und Qualität der Flüssigkeitsabgabe an.

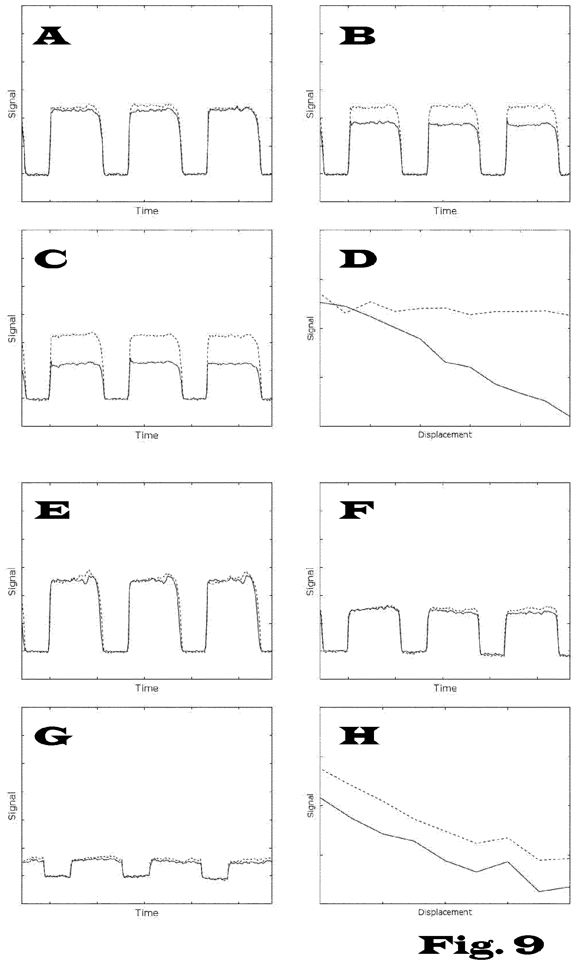

Figur 9 zeigt Sensorsignale eines Paars von Lichtschrankeneinheiten an einem Dosierkanal mit im Wesentlichen im rechten Winkel zu einander angeordneten Lichtstrahlen (Lichtschranke 1 = durchgehende Linie, Lichtschranke 2 = punktierte Linie). Messaufbau: Aufbau der Detektionseinheit auf einem in drei Raumrichtungen verstellbaren X-Y-Z-Tisch unterhalb eines Dosierkanals. Einstellung der Nadelposition zur optischen Achse erfolgte hier anhand des Schattenwurfs der Dosiernadel: Nadel hing zunächst in die Lichtschranke hinein - im Sensorsignal als Verringerung des Sensorsignals erkennbar - und wurde dann bis zur Oberkante des Lichtkegels der Lichtschranke hochgezogen. Die Signale zeigen jeweils eine dreimalig ausgelöste Flüssigkeitsabgabe bei idealer zentrischer Durchquerung des Lichtstrahls (Figur 9A) und bei Verschiebung der Flüssigkeitsstrahlen aus der Idealposition, das heißt bei Fehldosierungen (Figuren 9B und C). Figur 9D zeigt die Abhängigkeit der Signalamplitude von der der Position oder Ablenkung des Flüssigkeitsstrahls. Die Figuren 9A bis D zeigen die Situation bei Verschiebung des Flüssigkeitsstrahls etwa 90°, das heißt quer zum Lichtstrahl der ersten Lichtschrankeneinheit und etwa 0°, das heißt entlang der optischen Achse Lichtstrahls der zweiten Lichtschrankeneinheit. Obwohl der Flüssigkeitsstrahl streng entlang der Längsachse eines Lichtstrahls der zweiten Lichtschrankeneinheit verschoben wird, kommt es, wie Figur 9D zeigt, auch zu einem Abfall der Signalamplitude in der zweiten Einheit (siehe gestrichelte Linie). Figuren 9E bis H zeigen denselben Versuchsablauf bei diagonaler Verschiebung Ablenkung der Flüssigkeitsstrahlen etwa 45° zum Lichtstrahl der ersten Lichtschrankeneinheit und etwa 45° zum Lichtstrahl der zweiten Lichtschrankeneinheit..

Figur 10 zeigt gemessene elektrische Signale analog zu Figur 9. In Figur 10A ist ein im Wesentlichen ungestörter idealer Strahlverlauf intermittierend abgegebener Flüssigkeitsstrahlen gezeigt. Figuren 10B bis C zeigen verschiedene "defekte" Dosierkanäle, die unterschiedlich stark "spritzen". Die Qualität, das heißt besonders Kontinuität und Volumenkonstanz der dosierten Flüssigkeit, nimmt in den Figuren 10B und C ab. Dies lässt auf eine Fehldosierung schließen. Figur 10 D zeigt die Abhängigkeit der Signalqualität, welche über die Signalvarianz ermittelt wird. Maßgeblicher Signalparameter zur Bewertung der Strahlqualität ist die Signalvarianz (in der Praxis: Rauschen auf den Peaks).

Figur 11 zeigt eine schematische Schrägansicht eines Ausschnitts der erfindungsgemäßen Sensorplatte nach Figur 6: Auf einem Träger (30), welcher gleichzeitig die elektrische Kontaktierung und die Leiterbahnen enthält, sind Lichtquellen (42) mit gegenüberliegenden Lichtsensoren (46) als SMD-Elemente aufgebracht. Der Träger (30) enthält weitere elektronische Bauelemente (38) zur Signalkonditionierung, die in SMD-Bauweise aufgebracht oder in Hybridbauweise in den Träger (30) integriert sind. Der Träger (30) enthält in der dargestellten Ausführung einen Trägerrahmen (34) mit jeweiligen Fenstern (32), wodurch die Flüssigkeitstropfen oder -Strahlen hindurchtreten können. Der Trägerrahmen (34) dient dabei zusätzlich als Apertur zur Strahlabschattung zur Verbesserung der Signalqualität am Sensor (46).

Figur 12 zeigt eine schematische Draufsicht auf die Anordnung gemäß Figuren 5, 6 und 11 einer einzigen Lichtschrankeneinheit, bestehend aus Lichtquelle (42) und gegenüberliegendem Sensor (46) mit Sensorfläche (48) und dazwischen liegenden Trägerrahmen (34) mit Fenster (32), durch welchen der Flüssigkeitstropfen oder - strahl (26) hindurchtreten kann. Der Trägerrahmen (34) dient dabei zusätzlich als Apertur zur Strahlabschattung zur Verbesserung der Signalqualität am Sensor (46). Die durch die Apertur erzeugte Strahlbreite (A) ist dort stets größer als die Breite (B) des durchtretenden Flüssigkeitstropfens oder -strahls (26). Maß A beschreibt die Blendenbreite. Diese ist ca. 2 bis 3 mal so groß wie der erwartete Strahl- oder Tropfendurchmesser (Maß B). Sensor (46) und LED-Lichtquelle (42) sind in einem Abstand von ca. 2 bis 4 mm angebracht. In einer bevorzugten Ausgestaltung als integrale Sensorplatte gemäß Figuren 6 und 11 für das I-DOT Mikrodosiersystem ist Maß A etwa 0,6 mm, in einer bevorzugten Ausgestaltung als integrale Sensorplatte gemäß Figuren 5A oder B für ein Multidrop-Mikrodosiersystem ist Maß A etwa 1 mm.

## Patentansprüche

1. Vorrichtung (10) zur optischen Kontrolle von an Multikanal-Mikrodosiereinrichtungen (20) intermittierend abgegebenen Flüssigkeitstropfen oder -strahlen (26), enthaltend:
mindestens eine jedem Dosierkanal (22) der Mikrodosiereinrichtung (20) zugeordnete und an diesem jeweils unmittelbar auf einem gemeinsamen Träger (30) angeordnete Lichtschrankeneinheit (40), jeweils bestehend aus:
Lichtquelle (42) mit inhomogenem Strahlprofil, und
Lichtsensor (46) mit Sensorfläche (48), auf welche der profilierte Lichtstrahl (44) der Lichtquelle (42) projiziert,
wobei der profilierte Lichtstrahl (44) jeweils quer zur Ausbreitungsrichtung der abgegebenen Flüssigkeitstropfen oder -strahlen (26) verläuft und breiter ist als der zu kontrollierende Flüssigkeitstropfen oder -strahl (26), **dadurch gekennzeichnet, dass** die Lichtquelle (42) und der Lichtsensor (46) so ausgebildet und ausgerichtet sind, dass der profilierte Lichtstrahl (44) ein kegelförmiges Lichtbündel ist mit der höchsten Intensität in der optischen Achse in der Mitte des Lichtbündels, wobei die Intensität zum Rand des Lichtbündels jeweils abfällt, und
dass der Lichtsensor (46) die Intensität des auftreffenden Lichtstrahls (44) über seine Ausdehnung in der Projektionsfläche integriert und sein Sensorstrom der integrierten Strahlintensität entspricht,
so dass die maximale Abschattung des Lichtstrahls (44) und Verminderung des Sensorstroms dann erfolgen kann, wenn der Flüssigkeitstropfen oder -strahl (26) den Lichtstrahl (44) gerade in dessen optischer Achse unterbricht.

2. Vorrichtung nach Anspruch 1, wobei jedem Dosierkanal (22) jeweils genau eine Lichtschrankeneinheit (40) zugeordnet ist.

3. Vorrichtung nach Anspruch 1, wobei jedem Dosierkanal (22) jeweils zwei Lichtschrankeneinheiten (40) zugeordnet sind, deren Lichtstrahl (44) zueinander in einem Winkel von 30° bis 150° stehen.

4. Vorrichtung nach einem der vorstehenden Ansprüche, weiter enthaltend:
programmierte Auswerteeinheit (60), die zumindest mit dem jeweiligen Lichtsensor (46) verbunden ist, zur Bestimmung der Intensität des auf die Sensorfläche (48) fallenden profilierten Lichtstrahls (44), und
Speichereinheit (62) und Recheneinheit (64) zur Ermittlung des zeitlichen Intensitätsverlaufs oder der Differenz der Intensität zum Zeitpunkt des Durchtritts des Flüssigkeitstropfens oder -strahls (26) und dem Zeitpunkt unmittelbar vor oder nach diesem Ereignis.

5. Vorrichtung nach Anspruch 4, weiter enthaltend:
Signal- und Steuereinrichtung (66) zur Signalisierung der Abweichung von einem vorbestimmbaren zeitlichen Intensitätsverlauf oder der Intensitätsdifferenz.

6. Vorrichtung nach Anspruch 4 oder 5, weiter enthaltend eine Signal-Rückleitung (68) verbunden mit einem Stellglied (28) an der Mikrodosiereinrichtung (20) zur Steuerung oder Regelung der Abgabe von Flüssigkeit aus dem jeweiligen Dosierkanal (22) der Mikrodosiereinrichtung (20).

7. Vorrichtung nach einem der vorstehenden Ansprüche, wobei die Lichtschrankeneinheiten (40) auf dem Träger (30) regelmäßig beabstandet und in Form eines Gitters (zweidimensionales Array) oder einer Reihe (eindimensionales Array) angeordnet sind und zusammen mit dem Träger (30) eine integrale Sensorplatte bilden.

8. Multikanal-Mikrodosiereinrichtung (20) zur steuerbaren intermittierenden Abgabe von Flüssigkeitstropfen oder -strahlen (26) enthaltend die optische Kontrollvorrichtung (10) nach einem der vorstehenden Ansprüche.

9. Verfahren zur kontrollierten Abgabe von Flüssigkeitstropfen oder -strahlen an Dosierkanälen einer Multikanal-Mikrodosiereinrichtung, enthaltend die Schritte:
- Auslösen einer kurzfristigen Abgabe jeweils eines einzelnen Flüssigkeitstropfens oder -strahls an den Dosierkanälen,
- Beleuchten des jeweils abgegebenen Flüssigkeitstropfens oder -strahls mit einem Lichtstrahl mit inhomogenem Strahlprofil, wobei der profilierte Lichtstrahl ein kegelförmiges Lichtbündel ist mit der höchsten Intensität in der optischen Achse in der Mitte des Lichtbündels, wobei die Intensität zum Rand des Lichtbündels jeweils abfällt, und
- Registrieren der jeweiligen Abschwächung der über die Breite des Lichtstrahls integrierten Intensität bei Abschattung des Lichtstrahls durch diesen Flüssigkeitstropfen oder -strahl an einem Lichtsensor, der die Intensität des auftreffenden Lichtstrahls über seine Ausdehnung in der Projektionsfläche integriert und dessen Sensorstrom der integrierten Strahlintensität entspricht, so dass die maximale Abschattung des Lichtstrahls und Verminderung des Sensorstroms dann erfolgen kann, wenn der Flüssigkeitstropfen oder -strahl den Lichtstrahl gerade in dessen optischer Achse unterbricht, und
- automatisches Bewerten des jeweils registrierten abgabekorrelierten Intensitätsverlaufs und
- Ausgeben eines Steuer- oder Warnsignals als Ergebnis der Bewertung zum Zwecke der Regelung der Abgabe der Flüssigkeit aus dem jeweiligen Dosierkanal und/oder zur Signalisierung von Fehldosierungen.

10. Verfahren nach Anspruch 9, mit der Maßgabe, dass die mittlere maximale Amplitude der abgabekorrelierten Intensitätsabschwächung bei Auslösen der Abgabe des einzelnen Flüssigkeitstropfens oder -strahls die Idealdosierung anzeigt, wohingegen
- die Verringerung der abgabekorrelierten Intensitätsabschwächung unter einen vorbestimmten Schwellwert,
- das Ausbleiben einer abgabekorrelierten Intensitätsabschwächung und/oder
- die Erhöhung der statistischen Signalvarianz eines abgabekorrelierten Intensitätsverlaufs über einen vorbestimmten Schwellwert
jeweils eine Fehldosierung an dem jeweiligen Dosierkanal anzeigt.

## Claims

1. A device (10) for the optical control of liquid drops or spurts (26) which are intermittently dispensed from multichannel microdispensing devices (20), including:
at least one light barrier unit (40) assigned to each dosing channel (22) of the microdispensing device (20), that is in each case arranged directly thereto on a common carrier (30), each consisting of:
a light source (42) having an inhomogeneous beam profile and
a light sensor (46) having a sensor surface (48) onto which the profiled light beam (44) of the light source (42) projects,
wherein the profiled light beam (44) extends in each case transversely to the direction of propagation of the emitted liquid drops or spurts (26) and is wider than the liquid drop or spurt (26) to be controlled, **characterised in that** the light source (42) and the light sensor (46) are constructed and aligned in such a way
that the profiled light beam (44) is a cone-shaped light bundle with the highest intensity in the optical axis in the center of the light bundle, the intensity decreasing towards the edge of the light bundle, and
**in that** the light sensor (46) integrates the intensity of the incident light beam (44) over its extent in the projection surface and its sensor current corresponds to the integrated beam intensity,
so that the maximum shading of the light beam (44) and reduction of the sensor current can occur when the liquid drop or spurt (26) interrupts the light beam (44) just in its optical axis.

2. The device according to claim 1, wherein in each case precisely one light barrier unit (40) is assigned to each dosing channel (22).

3. The device according to claim 1, wherein two light barrier units (40) are assigned to each dosing channel (22), their light beams (44) beeing at an angle of 30° to 150°.

4. The device according to any one of the preceding claims, further including:
a programmed evaluation unit (60), which is at least connected to the respective light sensor (46) for determining the intensity of the profiled light beam (44) as falling on the sensor surface (48), and
a storage unit (62) and a computing unit (64) for determining the temporal course of intensity or the difference in intensity at the time the liquid droplet or spurt (26) is passing and at the instance immediately before or after this event.

5. The device according to claim 4, further including:
signal and control means (66) for signaling the deviation from a predeterminable temporal course of intensity or from the intensity difference.

6. The device according to claim 4 or 5, further including a signal return line (68) connected to an actuator (28) on the microdispensing device (20) for controlling or regulating the dispensing of liquid from the respective dosing channel (22) of the microdispensing device (20).

7. The device according to any one of the preceding claims, wherein the light barrier units (40) are spaced periodically on the carrier (30) and are arranged in the form of a grid (two-dimensional array) or a row (one-dimensional array) and form an integral sensor plate together with the carrier (30).

8. A multi-channel microdispensing device (20) for the controllable intermittent dispensing of liquid drops or spurts (26), including the optical control device (10) according to any one of the preceding claims.

9. A method for the controlled dispensing of liquid drops or spurts on dosing channels of a multichannel microdispensing device, including the steps of:
- triggering a short-term dispensing of a single liquid droplet or spurt at the dosing channels,
- illuminating the respectively dispensed liquid droplet or spurt with a light beam having an inhomogeneous beam profile, wherein the profiled light beam is a cone-shaped light bundle with the highest intensity in the optical axis in the center of the light bundle, the intensity decreasing towards the edge of the light bundle and
- registering the respective attenuation of the intensity integrated over the width of the light beam when the light beam is shaded by this liquid drop or spurt at a light sensor which integrates the intensity of the incident light beam over its extension in the projection surface and whose sensor current corresponds to the integrated beam intensity, so that the maximum shading of the light beam and reduction of the sensor current can occur when the liquid drop or spurt interrupts the light beam just in its optical axis, and
- automatic evaluation of the respectively registered dispensing-correlated intensity course, and
- outputting a control or warning signal as a result of the evaluation for the purpose of regulating the dispensing of the liquid from the respective dosing channel and/or for signaling of incorrect dosing.

10. The method according to claim 9, providing that the maximum mean amplitude of the dispensing-correlated attenuation of intensity upon initiation of the single liquid drop or spurt indicates the ideal dosage, whereas
- the reduction of the dispensing-correlated attenuation of intensity to below a predetermined threshold,
- the absence of a dispensing-correlated attenuation of intensity and/or
- the increase in the statistical signal variation of a dispensing-correlated intensity course to over a predetermined threshold value
in each case indicates an incorrect dosing at the respective dosing channel.

## Revendications

1. Dispositif (10) pour le contrôle optique de gouttes ou de jets de liquide (26) délivrés par intermittence à des dispositifs de microdosage à canaux multiples (20), contenant :
au moins une unité de barrière lumineuse (40) associée à chaque canal de dosage (22) du dispositif de microdosage (20) et disposée directement sur celui-ci sur un support commun (30), chacune consistant en :
une source de lumière (42) avec un profil de rayon inhomogène, et
capteur de lumière (46) avec une surface de capteur (48) sur laquelle se projette le rayon lumineux profilé (44) de la source de lumière (42),
dans lequel le rayon lumineux profilé (44) s'étend à chaque fois transversalement à la direction de propagation de gouttes ou de jets de liquide (26) émis et est plus large que la goutte ou le jet de liquide (26) à contrôler, **caractérisé en ce que** la source de lumière (42) et le capteur de lumière (46) sont construits et orientés de telle manière
que le rayon lumineux profilé (44) est un faisceau lumineux de forme conique avec l'intensité la plus élevée dans l'axe optique au centre du faisceau lumineux, l'intensité diminuant vers le bord du faisceau lumineux, respectivement, et
**en ce que** le capteur de lumière (46) intègre l'intensité du rayon lumineux incident (44) sur son étendue dans la surface de projection et son courant de capteur correspond à l'intensité intégrée du rayon,
de sorte que l'ombrage maximal du rayon lumineux (44) et la réduction du courant de capteur peuvent se produire lorsque la goutte ou le jet de liquide (26) interrompt le rayon lumineux (44) juste dans son axe optique.

2. Dispositif selon la revendication 1, dans lequel chaque canal de dosage (22) est associé à exactement une unité de barrière lumineuse (40).

3. Dispositif selon la revendication 1, dans lequel deux unités de barrière lumineuse (40) sont attribuées à chaque canal de dosage (22), dont le rayon lumineux (44) forment un angle de 30° à 150° l'un par rapport à l'autre.

4. Dispositif selon l'une des revendications précédentes, contenant en outre :
une unité d'évaluation programmée (60), qui est reliée au moins au capteur de lumière respectif (46), pour déterminer l'intensité du rayon lumineux profilé (44) tombant sur la surface de capteur (48), et
Unité de mémoire (62) et unité de calcul (64) pour déterminer l'évolution temporelle de l'intensité ou la différence d'intensité au moment du passage de la goutte ou du jet de liquide (26) et le moment immédiatement avant ou après cet événement.

5. Dispositif selon la revendication 4, contenant en outre :
des moyens de signalisation et de commande (66) pour signaler l'écart par rapport à une courbe d'intensité temporelle prédéterminable ou la différence d'intensité.

6. Dispositif selon la revendication 4 ou 5, contenant en outre une ligne de retour de signal (68) connectée à un actionneur (28) sur le dispositif de microdosage (20) pour commander ou réguler la distribution de liquide à partir du canal de dosage respectif (22) du dispositif de microdosage (20).

7. Dispositif selon l'une quelconque des revendications précédentes, dans lequel les unités de barrière lumineuse (40) sont régulièrement espacées sur le support (30) et disposées sous la forme d'une grille (réseau bidimensionnel) ou d'une rangée (réseau unidimensionnel) et forment avec le support (30) une plaque de détection intégrale.

8. Dispositif de microdosage à canaux multiples (20) pour la distribution intermittente contrôlable de gouttes ou de jets de liquide (26) contenant le dispositif de contrôle optique (10) selon l'une quelconque des revendications précédentes.

9. Procédé pour la distribution contrôlée de gouttes ou de jets de liquide au niveau des canaux de dosage d'un dispositif de microdosage à canaux multiples, contenant les étapes suivantes :
- Déclenchement d'une distribution de courte durée d'une seule goutte ou d'un seul jet de liquide dans chacun des canaux de dosage,
- illuminer la goutte ou le jet de liquide distribué respectif avec un rayon lumineux ayant un profil de rayon inhomogène, le rayon lumineux profilé étant un faisceau lumineux de forme conique avec l'intensité la plus élevée dans l'axe optique au centre du faisceau lumineux, l'intensité diminuant vers le bord du faisceau lumineux, et
- enregistrer l'atténuation respective de l'intensité intégrée sur la largeur du rayon lumineux lorsque le rayon lumineux est ombragé par cette goutte ou ce faisceau de liquide au niveau d'un capteur de lumière qui intègre l'intensité du rayon lumineux incident sur son extension dans la surface de projection et dont le courant de capteur correspond à l'intensité intégrée du rayon, de sorte que l'ombrage maximal du rayon lumineux et la réduction du courant de capteur peuvent avoir lieu lorsque la goutte ou le faisceau de liquide interrompt le faisceau lumineux juste dans son axe optique, et
- évaluer automatiquement la courbe d'intensité corrélée à la distribution enregistrée respective, et
- émettre un signal de commande ou d'avertissement à la suite de l'évaluation, afin de réguler la distribution du liquide à partir du canal de dosage respectif et/ou de signaler un dosage incorrect.

10. Procédé selon la revendication 9, à condition que l'amplitude maximale moyenne de l'atténuation d'intensité corrélée à la distribution lors du déclenchement de la distribution de la goutte ou du jet de liquide individuel indique le dosage idéal, alors que
- la réduction de l'atténuation de l'intensité corrélée à la distribution en dessous d'un seuil prédéterminé,
- l'absence d'atténuation de l'intensité corrélée à la distribution, et/ou
- l'augmentation de la variance du signal statistique d'une courbe d'intensité corrélée à la distribution au-dessus d'une seuil prédéterminée
indique dans chaque cas une erreur de dosage au niveau du canal de dosage respectif.
